# FASCICULE DE BREVET EUROPEEN

(11) **EP 2 247 698 B1**
(45) Date de publication et mention de la délivrance du brevet: **14.11.2012**
(21) Numéro de dépôt: 08857003.1
(22) Date de dépôt: 18.09.2008
(51) Int. Cl.: C10L 1/02

(54) **PROCÉDÉ DE TRANSFORMATION DE BIOMASSE EN PRODUITS RENFERMANT DES GROUPEMENTS ACÉTALS ET LEUR UTILISATION COMME BIOCARBURANTS.**
VERFAHREN ZUR UMWANDLUNG VON BIOMASSE IN PRODUKTE MIT ACETALGRUPPEN UND DEREN VERWENDUNG ALS BIOTREIBSTOFFE
METHOD FOR CONVERTING BIOMASS INTO PRODUCTS CONTAINING ACETAL GROUPS AND USE THEREOF AS BIOFUELS

(30) Priorité: 21.09.2007 FR 0706653
(43) Date de publication de la demande: 10.11.2010
(73) Titulaire: IFP Energies nouvelles, 92852 Rueil-Malmaison Cedex (FR)
(72) Inventeur: DELFORT, Bruno, F-75005 Paris (FR); MARCHAL, Rémy, F-78400 Chatou (FR)
(86) Numéro de dépôt international: PCT/FR2008/001309
(87) Numéro de publication internationale: WO 2009/071769

(56) Documents cités:
- WO-A-00/17290
- WO-A-2005/010131
- FR-A- 2 497 223
- FR-A- 2 544 738
- FRUSTERI ET AL: "Oxygenated additives production for diesel engine emission improvement" CHEMICAL ENGINEERING JOURNAL, ELSEVIER SEQUOIA, LAUSANNE, CH, vol. 134, no. 1-3, 30 août 2007 (2007-08-30), pages 239-245, XP022223951 ISSN: 1385-8947

## Description

### Domaine de l'invention :

La présente invention s'inscrit dans le cadre du développement actuel des bioraffineries. Elle concerne un procédé de transformation de biomasse en produits renfermant des groupements acétals et leur utilisation dans le pool gazole.

Ainsi la présente invention consiste en la fermentation de matières premières renouvelables d'origine végétale suivie de leur transformation en produits qui sont des substituts de produits issus aujourd'hui majoritairement de la transformation de matières premières d'origine fossile comme les carburants ou les produits fabriqués par l'industrie chimique.

Plus précisément, l'invention consiste à transformer des produits issus dé différents types de fermentations en une espèce chimique de la famille des acétals qui sera ensuite avantageusement utilisée comme biocarburant(s) ou incorporée à des carburants ou à des biocarburants ou à des compositions les renfermant.

### Examen de l'art antérieur :

La bioraffinerie constitue un élément clef d'une gestion raisonnée des bioressources. Lors du congrès de Rio de Janeiro en juin 1992, 170 états ont intégré le principe de gestion raisonnée dans leur programme d'action pour le vingt et unième siècle ; en septembre 2002, ce principe a même été réaffirmé lors du sommet mondial de Johannesburg. La bioraffinerie est, selon la définition de l'US Department of Energy, un concept global d'installation dans lequel la ressource de biomasse est extraite puis convertie en une large gamme de produits valorisables. Le schéma logique de la bioraffinerie est emprunté à la pétrochimie. Les composés intermédiaires (parfois dénommés synthons) dérivés des sucres y occupent une place centrale puisque ce sont eux qui conduisent aux produits finals qui seront délivrés sur le marché (Ohara et al., Appl. Microbiol. Biotechnol. 62, 474-477 (2003) ; Kamm and Kamm, Appl. Microbiol. Biotechnol. 62, 137-145 (2004)). Les alcools et les cétones font partie de ces synthons importants obtenus à partir de biomasse.

Dans une bioraffinerie, les flux d'alcools et de composés contenant un groupement carbonyle tel que principalement l'acétone peuvent provenir de la distillation des moûts de fermentation ou de leur séparation au moyen de membranes.

Parmi les différents types de fermentation bien connus de l'homme du métier et qui utilisent des matières premières renouvelables d'origine végétale, on peut citer :
- La fermentation acétonobutylique ou ABE (acétone-butanol-éthanol) qui met principalement en oeuvre l'espèce bactérienne *Clostidium acetobutylicum.* La fermentation ABE produit deux grandes familles de composés, les alcools (éthanol, butanol) d'une part et un composé carbonylé, l'acétone, d'autre part (Spivey M.J. (1978) : The acetone/ butanol/ethanol fermentation. Process Biochem 13:2-25). Dans les moûts de fin de culture, les alcools sont communément obtenus à une concentration comprise entre 150 et 190 mM tandis que la concentration finale d'acétone est comprise entre 85 et 170 mM.
- La fermentation IBE (isopropanol-butanol-éthanol) qui met principalement en oeuvre les souches de *Clostridium bejerinckii,* classées antérieurement en tant que *Clostridium butylicum*. La fermentation IBE produit trois alcools principaux (éthanol, butanol et isopropanol) ainsi que de l'acétone. Les concentrations finales en alcools de la fermentation IBE sont voisines de celles de la fermentation ABE. Toutefois, la concentration en acétone est généralement plus faible que pour la fermentation ABE.
- La fermentation éthanolique. Dans une bioraffinerie, l'éthanol provient en général de la fermentation alcoolique conduite avec la levure *Saccharomyces cerevisiae*. Il est cependant possible d'utiliser d'autres micro-organismes et notamment la bactérie *Zymomonas mobilis.* Avec *Saccharomyces cerevisae* et les espèces du genre *Saccharomyces,* la teneur finale d'éthanol est voisine d'une centaine de grammes par litre de milieu.

Les substrats des fermentations ABE et IBE sont soit des sucres simples, monomères ou dimères tels que le glucose et le saccharose, respectivement, soit encore des sucres complexes tels que l'amidon. Les principales matières premières amylacées sont les céréales (blé, maïs, orge...) et la pomme de terre. A partir des matières amylacées, les sucres monomériques peuvent être obtenus par hydrolyse avec des amylases commerciales. Cependant, de nombreuses souches de *Clostridium acetobutylicum* et *Clostridium bejerinckii* sont capables d'utiliser directement l'amidon. Avec ces souches, on peut donc se dispenser de l'hydrolyse préalable de l'amidon. De façon similaire aux substrats amylacés, les substrats lignocellulosiques peuvent être hydrolysés par des hydrolases commerciales (cellulases) pour fournir des sucres monomères en C6 (glucose principalement) et C5 (xylose et arabinose). *Clostridium acetobutylicum* et *Clostridium bejerinckii* sont capables d'utiliser aussi bien les sucres monomères en C5 qu'en C6.

Classiquement, les produits issus de la fermentation sont dirigés vers le pool essence. En effet, le mélange ABE présente un indice d'octane élevé et en mélange avec l'essence, il n'entraîne pas de problèmes de stabilité en présence de traces d'eau, ni de phénomène de démixtion. Son indice de cétane est trop faible pour pouvoir être avantageusement incorporé au pool gazole.

Le document FR 2 544 738 décrit l'utilisation de composés de type acétals, obtenu à partir de butanol préparé à partir de réactifs issus eux-mêmes de la biomasse, comme carburant pour moteurs Diesel.

On a découvert que si l'on combine astucieusement les différents flux issus des unités de fermentation citées précédemment, selon une réaction chimique d'acétalisation mettant en jeu au moins un alcool avec au moins un composé contenant un groupement carbonyle tels les cétones et plus précisément l'acétone selon le schéma général illustré plus loin, on pouvait obtenir un acétal ou un mélange d'acétals, compatible et soluble avec des carburants gazole ou des biocarburants et utilisables dans des moteurs Diesel.

### Présentation sommaire de l'invention:

L'invention décrit un procédé de transformation de biomasse en produits incorporables au pool gazole. Selon la revendication 1.

Le procédé selon l'invention comprend donc une étape (c) au cours de laquelle le mélange issu de l'étape (b) est incorporé à une base gazole et/ou à un biodiesel.

### Présentation détaillée de l'invention :

Le procédé selon la présente invention présente l'avantage d'utiliser des charges provenant de matières premières renouvelables d'origine végétale dans le cadre d'une bioraffinerie. L'ensemble des réactifs nécessaires pour la réaction d'acétalisation et la fabrication d'acétals compatibles avec les carburants est directement obtenu lors de la première étape de fermentation des produits ex-biomasse. La totalité des co-produits issus de la fermentation est ainsi utilisée pour la mise en oeuvre de la réaction d'acétalisation.

Les produits obtenus par le procédé selon la présente invention peuvent être utilisés comme carburants ou incorporés à une base carburant. Ainsi, il est possible de diriger des produits issus directement de la biomasse vers le pool gazole.

Les matières premières d'origine végétale subissent une première étape de fermentation. Ces matières premières peuvent être issues de plantes sucrières (betterave, canne à sucre), de plantes amylacées (maïs, blé, orge, pomme de terre...) ou de lignocellulose (résidus agricoles, forestiers, taillis à courte rotation...).

Les matières premières peuvent être soumises, préalablement à l'étape (a), à un prétraitement et/ou hydrolyse, étapes parfois nécessaires pour en extraire les sucres fermentescibles en C5 et C6. Pour les matières premières lignocellulosiques, l'hydrolyse est habituellement précédée d'un traitement qui rend la cellulose et les hémicelluloses accessibles aux hydrolases. Les principaux traitements avant hydrolyse sont les prétraitement alcalins (à la soude, par exemple, analogue à celui utilisé en papeterie), les prétraitements acides (acide sulfurique entre 1 et 3%) à des durées et températures variables et les prétraitements à la vapeur appelés encore hydrolyse flash ou "steam-explosion". Dans le prétraitement à la vapeur, une autohydrolyse s'effectue sous l'effet des groupements acétyles libérés à partir des nombreux sucres substitués des hémicelluloses, opérant ainsi, *in situ*, une hydrolyse partielle de ces polymères. Le prétraitement à la vapeur entraîne également la fusion des lignines suivie de leur recondensation en gouttelettes lors du refroidissement ainsi qu'une déstructuration intense du matériau lors de la libération explosive du produit. Ces trois effets combinés contribuent à une augmentation très sensible de la susceptibilité enzymatique du végétal prétraité à la vapeur. Un quatrième type de prétraitement est le prétraitement aux solvants tels qu'à l'éthanol à chaud.

Les matières premières d'origine végétale sont ensuite soumises à l'étape proprement dite de fermentation, qui peut être de trois types :
- fermentation ABE et/ou
- fermentation IBE
- et fermentation éthanolique.

Les fermentations de type ABE ou IBE sont en effet indispensables dans le procédé selon la présente invention puisqu'elles permettent d'obtenir les produits nécessaires à la réaction d'acétalisation. Elles sont complétées par une fermentation de type éthanolique, permettant d'obtenir ainsi un flux plus important d'alcool(s).

A l'issue de cette étape de fermentation, on récupère différents flux d'alcools en fonction du type de fermentation ayant eu lieu et des flux de composés contenant un groupement carbonyle. Dans le cas des fermentations ABE et IBE, on obtient aussi de faibles quantités d'acides carboxyliques (acétique et butyrique) ainsi que des gaz (hydrogène et CO₂)

Ces différents flux (alcools et composés contenant un groupement carbonyle) peuvent provenir de la distillation des moûts de fermentation ou de leur séparation au moyen de membranes.

Les alcools obtenus sont essentiellement de l'éthanol, du n-butanol ou de l'isopropanol. Le composé contenant un groupement carbonyle est principalement de l'acétone provenant de fermentation de type ABE et/ou IBE.

Ces différents flux sont mélangés et constituent les réactifs nécessaires à la réaction de formation d'au moins un acétal.

En effet, la réaction générale de synthèse d'acétals peut s'écrire : 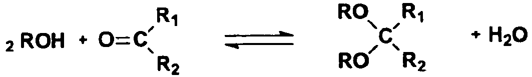 où ROH représente un alcool choisi principalement parmi l'éthanol, l'isopropanol, le n-butanol ... et où R₁ et R₂ représentent des atomes d'hydrogène ou des radicaux alkyles tels des groupes méthyles. Lorsque R1 = R2 = CH₃, le composé carbonylé est de l'acétone et on peut écrire la réaction :

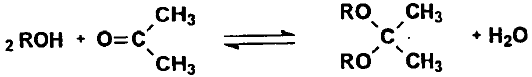

Lorsque que l'on met en jeu un mélange de plusieurs alcools la réaction devient par exemple dans le cas d'un mélange de deux alcools 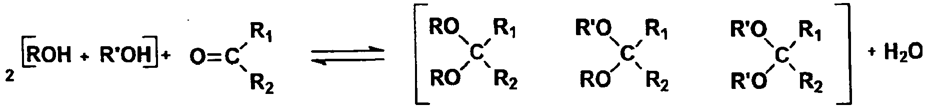 dans laquelle ROH et R'OH représentent chacun un alcool choisi parmi principalement l'éthanol, l'isopropanol, le n-butanol ...
Lorsque R1 = R2 = CH₃ le composé carbonylé est de l'acétone et on peut écrire la réaction ainsi :

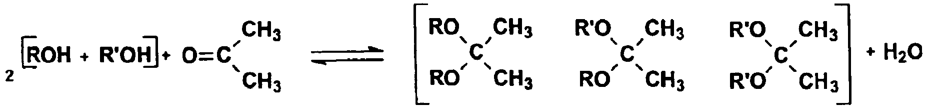

Le nombre d'alcools différents et de composés carbonylés différents mis en jeu dans la réaction d'acétalisaition n'est pas limitatif.

On peut citer parmi les acétals ainsi formés selon l'invention le 2,2-diéthoxy propane, le diisopropoxy-2,2-propane, le di-n-butoxy-2,2-propane, le éthoxy-2-isopropoxy-2-propane, le éthoxy-2-n-butoxy-2-propane, le n-butoxy-2-isopropoxy-2-propane.

Cette réaction générale de formation d'acétals est une réaction bien connue en chimie organique. Il s'agit d'une réaction équilibrée mettant en jeu 2 moles d'alcool pour une mole de cétone ou d'aldéhyde. La réaction produit une molécule d'acétal et une molécule d'eau. Il s'agit d'une réaction équilibrée qui peut être favorisée en utilisant un excès de l'un des réactifs ou en éliminant l'eau de condensation pendant la réaction.
Afin de déplacer l'équilibre dans le sens favorable à la formation d'acétal, on utilise généralement un excès d'alcool par rapport à la stoechiométrie. Le rapport molaire entre le nombre de moles d'alcool(s) et celui de la cétone ou de l'aldéhyde est compris entre 2,1 et 20 et préférentiellement entre 2,2 et 6. On peut également favoriser la réaction en éliminant l'eau de condensation formée pendant la réaction. Pour cela, on peut par exemple opérer en présence de tamis moléculaire.
Parfois on a recours simultanément à ces deux principes.

La température de réaction est comprise entre la température ambiante et 180°C. Plus généralement, lorsqu'on utilise de l'acétone, on effectue la réaction à 56°C, ce qui correspond à la température d'ébullition de l'acétone à la pression atmosphérique. On peut également opérer à une température supérieure à une pression supérieure à la pression atmosphérique en utilisant un équipement de type autoclave permettant d'opérer sous pression.

La réaction de formation d'acétals à partir d'alcools et de cétones ou d'aldéhydes est généralement catalysée par les acides dont la concentration peut être comprise entre 0,1 et 5% en mole par rapport au nombre de moles de composés contenant un groupement carbonyles, de préférence entre 0,3 et 1,5% en mole.
Parmi les catalyseurs les plus utilisés, on peut citer sans être exhaustif l'acide sulfurique, l'acide chlorhydrique, l'acide paratoluènesulfonique ainsi que des catalyseurs acides de type hétérogènes tels les résines sulfoniques comme par exemple certaines résines Amberlyst™ notamment l'Amberlyst 15.

Le mécanisme de la réaction est un mécanisme cationique dont le détail est présenté ci-après.

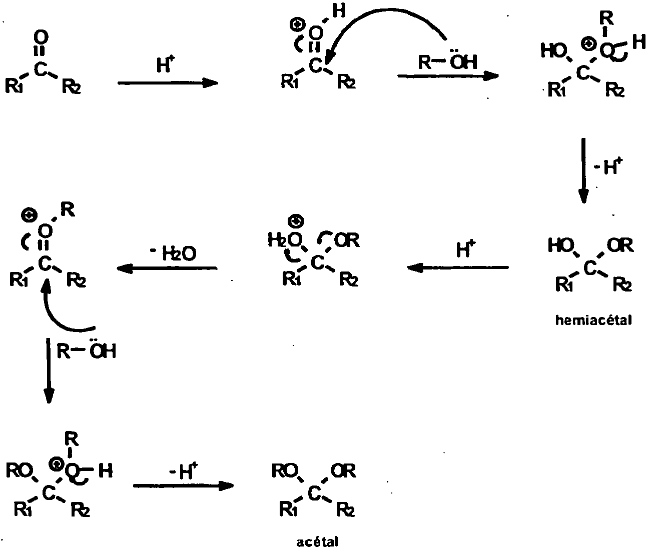

L'addition d'une première molécule d'alcool sur une fonction carbonyle conduit à la formation d'un hémiacétal. La formation d'un hémiacétal est réversible et l'intermédiaire de cette réaction est un ion oxonium. La réaction commence avec la protonation de l'oxygène du carbonyle suivie de la perte d'une molécule d'eau. La plupart des hémiacétals sont instables par rapport aux composés carbonylés dont ils sont issus (les hémiacétals qui se forment par cyclisation intramoléculaire sont par contre souvent stables si le cycle formé comporte cinq ou six atomes). En milieu acide, les hémiacétals peuvent subir une protonation suivie d'une réaction d'élimination d'eau qui conduit à un ion oxonium non protoné instable, très réactif vis-à-vis d'une deuxième molécule d'alcool et qui conduit après la perte d'un proton à la formation d'un acétal. En fait, chaque étape de formation d'un acétal est réversible.

On peut également préparer des acétals par réaction entre un alcool et un acétal déjà existant. Il s'agit d'une réaction d'échange d'alcool à partir d'un acétal appelée transacétalisation. Cette réaction catalysée par les acides est réversible. Elle est favorisée par l'élimination en continu de l'alcool partant.

La préparation d'acétal ou de mélange d'acétals peut être réalisée eh batch dans un réacteur agité ou en continu dans un réacteur de type piston ou tubulaire renfermant un catalyseur solide hétérogène.

La réaction d'acétalisation est suivie d'une étape de séparation permettant d'éliminer l'eau et les réactifs en excès.
On obtient ainsi un mélange d'acétals que l'on peut directement envoyer vers le pool carburant de la bioraffinerie.

L'ensemble du procédé selon l'invention peut être résumé par le schéma de principe suivant :

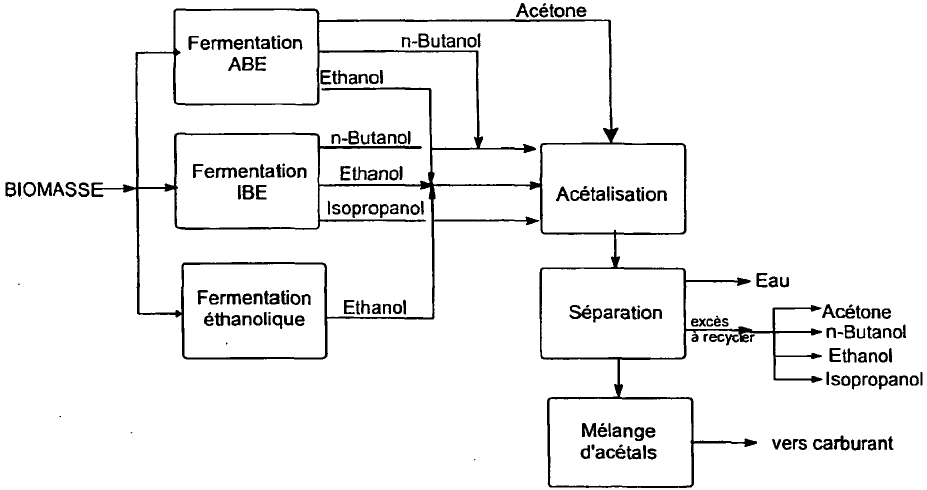

Il est également envisageable de compléter le flux d'acétone issu des différentes fermentations par une autre source extérieure de composés carbonylés.

### Exemples :

### Exemple 1 : production d'acétone, de butanol et d'éthanol à partir de substrats lignocellulosiques

Le substrat utilisé est la rafle de maïs. La rafle (5,5 tonnes) est prétraitée à la vapeur en continu dans une machine STAKE à la pression de 13 à 14 bar pendant 3 min. La biomasse prétraitée, séparée en deux lots équivalents, est soumise à l'hydrolyse enzymatique dans un réacteur de 25 m³ pendant 24 heures avec des cellulases de *Trichoderma reseii* CL 847, à raison de 30 UFP (unités "papier filtre") par gramme de matière sèche: A la fin de l'opération et après avoir rassemblé les deux lots d'hydrolysats, les matières insolubles en suspension sont éliminées par centrifugation et on obtient alors 50 m³ d'un hydrolysat clarifié dont la composition en sucres est la suivante : Glucose 41,5 g/L, Xylose 23 g/L, arabinose 1 g/L.
L'hydrolysat de sucres est supplémenté avec 0.5 g/L KH₂PO₄, 0,2 g/L MgSO₄, 7H2O 10 mg FeSO4, 7H₂O et 3 g/L d'extrait de levure. Il est ensemencé avec une préculture de la souche *Clostridium acetobutylicum* NCIB 2951 à un taux d'ensemencement de 5 % (v/v) dans de conditions d'asepsie rigoureuses obtenues après stérilisation de l'hydrolysat à 110 °C pendant 40 min. En phase de croissance acidogène, le pH est régulé à 5,5 par addition d'ammoniaque. Après 50 heures de culture, on obtient un moût final qui renferme 7,8 g/L d'acétone, 12,4 g/L de butanol et 0,3 g/L d'éthanol, les gaz dégagés représentant 31 L/L de milieu de culture. Le moût de fermentation est envoyé en distillation après avoir été chauffé à 97 °C. Dans cette première étape de séparation, le butanol, l'acétone et 90 % d'éthanol sont entraînés à la vapeur. Le condensat contient environ 50 % (v/v) d'eau et 50 % de solvants. Celui-ci est envoyé à la distillation "ABE" où éthanol plus acétone est séparé d'eau plus butanol. Ensuite, la distillation "acétone-éthanol" sépare ces deux produits. Le résidu de la distillation ABE est décanté pour parvenir à la séparation du butanol et de l'eau. A l'issue de ce processus, on obtient 362 L d'acétone, 575 L de butanol et 12 L d'éthanol

### Exemple 2 : production d'éthanol à partir de substrats lignocellulosiques

L'hydrolysat de rafles tel qu'obtenu dans l'exemple 1 est supplémenté par KH₂PO₄ (3 g/L), (NH₄)₂SO₄ (2 g/L), MgSO₄, 7H₂O (0,5g/L), extrait de levure 1 (g/L). Il est fermenté avec la levure *Saccharomyces cerevisae* ATCC 2062. Après 12 heures de culture à la température de 28 °C au pH initial de 4.5, on obtient un moût final qui contient 20 g/L d'éthanol qui est récupéré par distillation.

### Exemple 3 : synthèse du di-n-butoxy-2.2-propane à partir des produits obtenus dans l'exemple 1

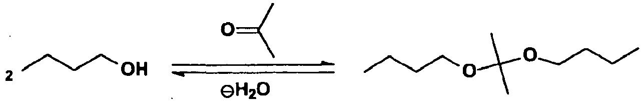

On introduit la totalité de la quantité de n-butanol préparé lors de l'exemple 1, c'est-à-dire 575 litres soit environ 465.7 kg (6294 moles) dans un réacteur équipé d'un système d'agitation, d'une colonne de reflux et d'un système de contrôle de la température incluant un système de refroidissement à l'eau par serpentin. On introduit ensuite 115 litres soit environ 91 kg (1573 moles) d'acétone prélevés dans la recette de l'exemple 1 ainsi que 3 kg de résine sulfonique Amberlyst 15, soit environ 14 équivalents molaires de fonction acide. On maintient le milieu sous agitation pendant une heure en contrôlant la température afin de demeurer à la température ambiante, puis on porte le milieu à reflux pendant 6 heures. Après retour à la température ambiante, on vidange le réacteur par gravitation à travers un filtre de manière à laisser le catalyseur solide dans le réacteur où il sera à nouveau utilisé pour une opération suivante. On dirige alors le mélange obtenu vers une colonne de distillation où l'on procède à une séparation de l'acétone résiduelle, de l'excès de n- butanol et de l'eau de condensation dans des conditions conventionnelles bien connues de l'homme du métier. On récupère 271 kg d'un liquide qui correspond à du di-n-butoxy-2.2-propane.

### Exemple 4 : synthèse d'un mélange d'acétals à partir des produits obtenus dans les exemples 1 et 2

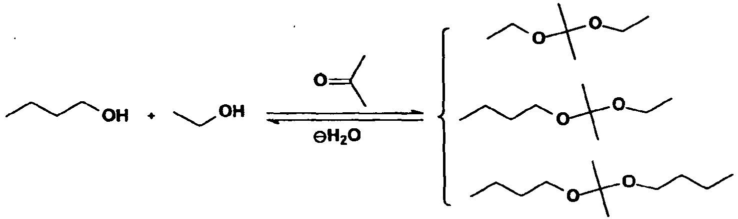

On introduit la totalité de la quantité de l'acétone préparée lors de l'exemple 1, c'est-à-dire 362 litres soit environ 286 kg (4937 moles) dans un réacteur équipé d'un système d'agitation, d'une colonne de reflux et d'un système de contrôle de la température incluant un système de refroidissement à l'eau par serpentin. On introduit ensuite la totalité de la quantité de n-butanol préparé lors de l'exemple 1, c'est-à-dire 575 litres soit environ 465.7 kg (6294 moles) ainsi que la totalité de la quantité d'éthanol préparé lors de l'exemple 1, c'est-à-dire 12 litres soit environ 15.2 kg (300 moles). A ce milieu on ajoute 350 litres soit environ 276 kg (6000 moles) d'éthanol préparé à partir de substrats lignocellulosiques dans les conditions décrites dans l'exemple 2. On introduit ensuite 8.5 kg de résine sulfonique Amberlyst 15, soit environ 41.6 équivalents molaires de fonction acide. On maintient le milieu sous agitation pendant deux heures en contrôlant la température afin de demeurer à la température ambiante, puis on porte le milieu à reflux pendant 7 heures. Après retour à la température ambiante, on vidange le réacteur par gravitation à travers un filtre de manière à laisser le catalyseur solide dans le réacteur où il sera à nouveau utilisé pour une opération suivante. On dirige alors le mélange obtenu vers une colonne de distillation où l'on procède à une séparation de l'acétone résiduelle, de l'excès d'éthanol, de l'excès de n-butanol et de l'eau de condensation dans des conditions conventionnelles bien connues de l'homme du métier. On récupère 761 kg d'un mélange d'acétals liquide dont la composition molaire est de 21% de diéthoxy-2.2-propane, 53% de éthoxy-2-n-butoxy-2-propane et 26% de di-n-butoxy-2.2-propane.

### Exemple 5

On réalise des mélanges d'un gazole de densité 0,832 à 15 °C, d'une teneur en soufre de 30 ppm et d'un indice de cétane de l'ordre de 53 avec respectivement 5%, 10% et 30% en volume du mélange d'acétals tel que préparé dans l'exemple 4. Les produits sont miscibles. Les indices de cétane des mélanges mesurés selon la méthode EN ISO 5165 sont respectivement de 53, 53 et 54 ce qui confirme la compatibilité des acétals préparés avec des carburants gazole.

### Exemple 6

On réalise le mélange d'un gazole de densité 0,832 à 15 °C, d'une teneur en soufre de 30 ppm et d'un indice de cétane de l'ordre de 53 , renfermant 5% en volume d'un ester méthylique d'huile de colza communément appelé biodiesel satisfaisant à la norme européenne EN14414 et 5% en volume du mélange d'acétals tel que préparé dans l'exemple 4. Les produits sont miscibles. L'indice de cétane du mélange mesuré selon la méthode EN ISO 5165 est de 53 ce qui confirme la compatibilité des acétals préparés avec des compositions de carburants gazole et de biodiesel.

### Exemple 7

On réalise le mélange d'un ester méthylique d'huile de colza communément appelé biodiesel satisfaisant à la norme européenne EN14414 avec 10% du mélange d'acétals tel que préparé dans l'exemple 4. Les produits sont miscibles. L'indice de cétane du mélange mesuré selon la méthode EN ISO 5165 est de 51 ce qui confirme la compatibilité des acétals préparés avec le biodiesel.

## Revendications

1. Procédé de transformation de biomasse en produits incorporables au pool gazole comprenant une première étape (a) de fermentation de type acétonobutylique et/ou IBE de matières premières renouvelables d'origine végétale à l'issue de laquelle on obtient au moins un alcool et au moins un composé contenant un groupement carbonyle, comprenant en outre une fermentation éthanolique et une deuxième étape (b) correspondant à une réaction d'acétalisation mettant en jeu au moins un alcool et au moins un composé contenant un groupement carbonyle obtenus dans l'étape (a).

2. Procédé selon la revendication 1 dans lequel les matières premières d'origine végétale sont issues de plantes sucrières, de plantes amylacées ou de plantes lignocellulosiques.

3. Procédé selon la revendication 1 ou 2 dans lequel les matières premières subissent un prétraitement et/ou hydrolyse préalablement à l'étape (a).

4. Procédé selon l'une des revendications précédentes dans lequel la réaction d'acétalisation de l'étape (b) est catalysée par des acides dont la concentration est comprise entre 0,1% et 5% en mole par rapport au nombre de moles de composé contenant un groupement carbonyle.

5. Procédé selon la revendication 4 dans lequel le catalyseur est choisi parmi l'acide sulfurique, l'acide chlorhydrique, l'acide paratoluènesulfonique ou les catalyseurs acides de type hétérogènes tels les résines sulfoniques.

6. Procédé selon l'une des revendications précédentes dans lequel l'étape (b) est réalisée à une température comprise entre la température ambiante et 180°C.

7. Procédé selon l'une des revendications 1 à 6 dans lequel l'étape (b) est réalisée en batch dans un réacteur agité.

8. Procédé selon l'une des revendications 1 à 7 dans lequel l'étape (b) est réalisée en continu dans un réacteur de type piston ou tubulaire.

9. Procédé selon l'une des revendications précédentes comprenant une étape (c) au cours de laquelle le mélange issu de l'étape (b) est incorporé à une base gazole et/ou à un biodiesel.

## Claims

1. A method of converting biomass to products that can be incorporated into the diesel fuel pool, comprising a first stage (a) of fermentation stage of acetonobutylic and/or IBE type of renewable raw materials of vegetable origin at the end of which a least one alcohol and at least one compound containing a carbonyl group are obtained, said first stage (a) further comprises an ethanolic fermentation, and a second stage (b) corresponding to an acetalization reaction involving at least one alcohol and at least one compound containing a carbonyl group obtained in stage (a).

2. A method as claimed in claim 1, wherein the raw materials of vegetable origin come from sugar plants, amylaceous plants or lignocellulosic plants.

3. A method as claimed in claim 1 or 2, wherein the raw materials are subjected to a pretreatment and/or hydrolysis prior to stage (a).

4. A method as claimed in any one of the previous claims, wherein the acetalization reaction of stage (b) is catalyzed by acids whose concentration ranges between 0.1 % and 5 % by mole in relation to the number of moles of compound comprising a carbonyl group.

5. A method as claimed in claim 4, wherein the catalyst is selected from among sulfuric acid, hydrochloric acid, paratoluenesulfonic acid or acid catalysts of heterogeneous type such as sulfonic resins.

6. A method as claimed in any one of the previous claims, wherein stage (b) is carried out at a temperature ranging between ambient temperature and 180°C.

7. A method as claimed in any one of claims 1 to 6, wherein stage (b) is carried out batchwise in a stirred reactor.

8. A method as claimed in any one of claims 1 to 6, wherein stage (b) is carried out on a continuous basis in a piston or tubular type reactor.

9. A method as claimed in any one of the previous claims, comprising a stage (c) wherein the mixture from stage (b) is incorporated into a diesel fuel base and/or a biodiesel fuel.

## Patentansprüche

1. Verfahren zur Umwandlung von Biomasse in Stoffe, die dem Dieselgemisch beigefügt werden können, wobei es einen ersten Schritt (a) der Aceton/Butanol- oder IBE-Vergärung erneuerbarer Rohstoffe pflanzlicher Herkunft umfasst, bei welchem mindestens ein Alkohol und mindestens eine carbonylgruppenhaltige Verbindung erhalten werden, wobei es darüber hinaus eine ethanolische Gärung und einen zweiten Schritt (b) umfasst, der einer Acetalbildungsreaktion entspricht, bei welcher mindestens ein Alkohol und mindestens eine carbonylgruppenhaltige Verbindung, wie sie in Schritt (a) erhalten wurden, zum Einsatz kommen.

2. Verfahren nach Anspruch 1, wobei die Rohstoffe pflanzlicher Herkunft aus Zuckerpflanzen, Stärkepflanzen oder lignocellulosehaltigen Pflanzen stammen.

3. Verfahren nach Anspruch 1 oder 2, wobei die Rohstoffe im Vorfeld des Schrittes a) eine Vorbehandlung und/oder Hydrolyse erfahren.

4. Verfahren nach einem der vorhergehenden Ansprüche, wobei die Acetalbildungsreaktion des Schrittes (b) durch Säuren katalysiert wird, deren Konzentration im Bereich von 0,1 % bis 5 Mol-% liegt, bezogen auf die Anzahl an Molen der carbonylgruppenhaltigen Verbindung.

5. Verfahren nach Anspruch 4, wobei der Katalysator aus Schwefelsäure, Salzsäure, para-Toluolsulfonsäure oder den sauren Katalysatoren heterogener Art wie etwa den Sulfonsäureharzen ausgewählt ist.

6. Verfahren nach einem der vorhergehenden Ansprüche, wobei der Schritt (b) bei einer Temperatur durchgeführt wird, die zwischen der Raumtemperatur und 180 °C einschließlich liegt.

7. Verfahren nach einem der Ansprüche 1 bis 6, wobei der Schritt (b) chargenweise in einem Rührkesselreaktor durchgeführt wird.

8. Verfahren nach einem der Ansprüche 1 bis 7, wobei der Schritt (b) kontinuierlich in einem Pfropfenströmungs- oder Rohrreaktor durchgeführt wird.

9. Verfahren nach einem der vorhergehenden Ansprüche, wobei es einen Schritt (c) umfasst, im Laufe dessen die Mischung, welche aus dem Schritt (b) stammt, einer Dieselgrundlage und/oder einem Biodiesel beigefügt wird.
